(19) Europäisches Patentamt
European Patent Office
Office européen des brevets

(11) Veröffentlichungsnummer: **0 413 958 A1**

# (12) EUROPÄISCHE PATENTANMELDUNG

(21) Anmeldenummer: **90113713.3**

(22) Anmeldetag: **18.07.90**

(51) Int. Cl.[5]: **C12N 9/22**

(30) Priorität: **26.07.89 DE 3924709**

(43) Veröffentlichungstag der Anmeldung:
**27.02.91 Patentblatt 91/09**

(84) Benannte Vertragsstaaten:
**AT BE CH DE DK ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Ankenbauer, Waltraud, Dr.**
**Flohbühlweg 2**
**D-8122 Penzberg(DE)**
Erfinder: **Jarsch, Michael, Dr. rer. nat.**
**Unterkarpfsee 11**
**D-8173 Bad Heilbrunn(DE)**
Erfinder: **Kessler, Christoph, Dr. Chem.**
**Schlossbergweg 11**
**D-8021 Dorfen(DE)**
Erfinder: **Laue, Frank**
**Seestrasse 16**
**D-8121 Pähl(DE)**

## (54) Typ II-Restriktionsendonuklease DsaV.

(57) Die TypII-Restriktionsendonuklease DsaV besitzt folgende Erkennungssequenz, spaltet bevorzugt an der durch die Markierung definierten Spaltstelle:

5'- |CCNGG -3'

3'- GGNCC| -5'

und ist aus Mikroorganismen der Gattung Dactylococcopsis erhältlich. Sie kann zur Erkennung und Spaltung der doppelsträngigen DNA-Sequenz CCNGG und der komplementären Sequenz eingesetzt werden.

EP 0 413 958 A1

Xerox Copy Centre

# TYP II-RESTRIKTIONSENDONUKLEASE DSAV

Die Erfindung betrifft die Typ II-Restriktionsendonuklease DsaV, ein Verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden in jedem Polynucleotidstrang einer Zielsequenz je eine Phosphodiesterbrücke hydrolysiert. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch besteht ein Bedarf an weiteren Restriktionsendonukleasen mit neuen Spezifitäten.

Gelöst wird diese Aufgabe erfindungsgemäß durch eine Typ-II-Restriktionsendonuklease mit der Erkennungssequenz und der durch die Markierung definierten Spaltstelle

| CCNGG

GGNCC |

Das Enzym ist bevorzugt aus Mikroorganismen der Gattung Dactylococcopsis erhältlich.

Die erfindungsgemäße neue Restriktionsendonuklease, die im folgenden als DsaV bezeichnet wird, hat ein Temperaturoptimum bei ca. 60° C. Das Enzym besitzt eine gute Aktivität zwischen pH 7,6 und pH 8,2 in 33 mmol/l Trisacetat Puffer mit 10 mmol/l $(CH_3COO)_2Mg$, 66 mmol/l $CH_3COOK$ und 0,5 mmol/l DTE (Dithioerythritol). Das pH-Optimum liegt bei ca. pH 7,9. DsaV hat die gleiche Erkennungssequenz wie ScrFI (Fitzgerald, G. F., Daly, C., Brown, L. R. and Gingeras, T. R. (1982) Necleic Acids Res. 10 , 8171 - 8179). ScrFI hat jedoch eine andere Spaltstelle, nämlich:

CC | N GG

GG N | CC

Die Erkennungssequenz läßt sich durch vollständigen Verdau der DNAs der Viren SV40 und Adeno 2, des Phagen Lambda und des Plasmids pUC18 bestätigen. Diese DNA-Moleküle werden mit DsaV behandelt.

Tabelle 1 zeigt einen Vergleich der experimentell beobachteten Schnittstellenspezifität mit einer computerermittelten Schnittstellenspezifität für ein Enzym, welches die Sequenz CCNGG erkennt.

Tabelle I:

| DNA | Experimentell bestimmte Fragmentlängen [bp] | Durch Computeranalyse bestimmte Fragmentlängen [bp] | Spaltpositionen ermittelt durch Computeranalyse bei den Basenpaaren | Anzahl der durch Computeranalyse ermittelte Spaltstellen |
|---|---|---|---|---|
| pUC18dcm⁻ | 700, 500, 350, 270/270, 220, 110/110, u.a. | 696, 501, 351, 288, 272, 218, 121, 110, 80, 35, 13, 1 | 135, 245, 246, 326, 598, 633, 1134, 1485, 2181, 2399, 2412, 2533 | 12 |
| SV 40 | 1000, 830, 680, 550, 450, 380, 320, 250, 200, 130/130, u.a. | 993, 823, 673, 552, 444, 369, 311, 249, 200, 127, 126, 101, 88, 61, 55, 54, 17 | 159, 176, 231, 357, 909, 963, 1407, 1534, 2527, 2896, 3145, 3246, 3334, 4007, 4068, 4891, 5091 | 17 |
| bp: Basenpaar(e) | | | | |

Die Spaltposition innerhalb der Erkennungssequenz des Enzyms läßt sich an einem M13-Derivat, das im Abstand von ca. 30 -200 Basen zur Bindungsstelle des universalen Sequenzierprimers (Messing, J. et al. (1981) Nucl. Acids Res. 9 , 309 -321) diese Erkennungssequenz trägt, bestimmen. An einzelsträngiger DNS des M13-Derivates werden zunächst mit dem universalen Sequenzierprimer Sequenzreaktionen nach der Didesoxy-Kettenabbruchmethode durchgeführt (Sanger, F., et al. (1977) Proc. Natl. Acad. Sci. USA 74 , 560 - 564, Messing, J. et al. (1981) Nucl. Acids Res. 9 , 309 - 321).

Parallel dazu wird der Sequenzierprimer mit T4-Polynukleotid-Kinase und [$\gamma$-$^{32}$P]ATP am 5′-Ende radioaktiv markiert. Nach Anhybridisieren dieses 5′-endmarkierten Sequenzierprimers an die einzelsträngige M13-DNS wird in einer Auffüllreaktion mit DNS-Polymerase I, Klenow-Fragment, und einer Desoxynukleotidtriphosphat-mischung aus dATP, dCTP, dGTP und dTTP eine partiell doppelsträngige DNS hergestellt. Ein Aliquot dieser DNS, deren neusynthetisierter Strang am 5′-Ende radioaktiv markiert ist, wird nun mit der Restriktionsendonuklease DsaV gespalten. Die Hälfte jedes Spaltansatzes wird zusätzlich noch mit T4-DNS-Polymerase in Gegenwart einer Mischung aller vier Desoxynukleotidtriphosphate behandelt, um glatte DNS-Enden zu erhalten.

Die Analyse der Reaktionsprodukte erfolgt durch Elektrophorese auf Sequenzgelen (8 mmol/l Harnstoff, 5 % Polyacrylamid) und anschließender Autoradiographie. Die Interpretation der Ergebnisse wird nach Brown, N.L. und Smith, M. (Methods in Enzymology 65 (1980) 391 - 401) durchgeführt. Durch Vergleich der Laufstrecken der radioaktiv markierten Fragmente mit der Sequenzleiter wird die Lage der Spaltstelle bestimmt. Die zusätzlich T4-DNS-Polymerase behandelten Proben zeigen im Vergleich zu den lediglich DsaV-gespaltenen Proben eine um fünf Basen verlängerte Ban de. Damit ist gezeigt, daß DsaV ein um fünf Basenpaare überhängendes 5′-Ende erzeugt.

Die Spaltung von DsaV erfolgt außerhalb der Erkennungssequenz daher mit folgender Spezifität:

```
5'...|CCNGG ...3'
     |_____
           |
3'... GGNCC|...5'
```

Die experimentell bestimmte Anzahl der Schnittstellen ist identisch mit der Anzahl der Schnittstellen, die durch Computeranalyse mit den verschiedenen DNAs für die Sequenz CCNGG erhalten wurde (Tabelle I). Zusätzlich wurden diese Daten noch mit den Tabellen aus Gene 10 (1980) 357 - 370, verglichen.

Erfindungsgemäß wird DsaV gewonnen, indem man Mikroorganismen der Gattung Dactylococcopsis, vorzugsweise Mikroorganismen der Art Dactylococcopsis salina züchtet und das Enzym aus den Zellen gewinnt. Besonders vorzugsweise wird Dactylococcopsis salina DSM 4080 verwendet. Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Lambda-dcm⁻-DNA. Die erhaltenen DNA-Fragmente werden in Agarosegelen in den für die Fragmentauftrennung üblichen Puffersystemen, in Gegenwart von Ethidiumbromid, elektrophoretisch aufgetrennt.

Die für die Gewinnung des Enzyms verwendeten Microorganismen wachsen aerob in einem Medium nach A.E. Walsky, J. van Rijn, Y. Cohen (1983), Proc. Royal Society London B217 , 417 -447.

Der Mikroorganismus wurde bei der Deutschen Sammlung von Mikroorganismen, Gesellschaft für biotechnologische Forschung mbH, Mascheroder Weg 16, 3300 Braunschweig, BRD, hinterlegt und trägt die Hinterlegungsnummer DSM 4080. Die optimalen Wachstumsbedingungen sind zwischen 20 und 30 °C, pH 6,5 - 7,5. Die Verdoppelungszeit beträgt etwa 3 Tage.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen in Tris/HCl Puffer, pH 8,0, welcher Protease-Inhibitoren enthält, suspendiert. Die Zellen werden über eine French-Presse aufgeschlossen und mit Polymin P präzipitiert. Die weitere Reinigung des Überstands wird vorzugsweise über Affinitätschromatographie, Ionentauscherchromatographie und Chromatographie über Hydroxylapatit durchgeführt. Als Material für die Affinitätschromatographie geeignet ist beispielsweise Heparin-Sepharose® CL-6B (Pharmacia).

Als Anionentauscher geeignet ist das unter dem Namen DEAE Sephacel® (Pharmacia) erhältliche Produkt. Als Kationenaustauscher ist Cellulose Phosphat P11 (Whatman) geeignet. Hydroxylapatit (BM) ist ebenfalls geeignet, aber auch andere Chromatographiematerialien, die dem Fachmann bekannt sind.

Die folgenden Beispiele erläutern die Erfindung weiter.

Beispiel 1

Dactylococcopsis salina DSM 4080 wird bei 21 °C im Aufgußverfahren 21 Tage in einem Glasfermenter, der kontinuierlich mit Quecksilberdampflampen bestrahlt wird, gezüchtet und in der späten logarithmischen bzw. stationären Phase geerntet. Als Kulturmedium werden 125 g NaCl, 10 g $MgCl_2.6\ H_2O$, 3,5 g $MgSO_4.7\ H_2O$, 2,5 g KCl, 0,75 g $NaNO_3$, 0,08 g $CaCl_2.2\ H_2O$, 7,8 mg $K_2HPO_4$, 20 mg $Na_2CO_3$, 3 mg Ammoniumeisen (III)-citrat, 3 mg Zitronensäure und 3 mg EDTA-$Na_2$ pro Liter verwendet, dem 1 ml folgender Spurenelementlösung (Konzentration für 1 Liter) zugesetzt wird: 2,86 g $H_3BO_3$, 1,81 g $MnCl_2.4\ H_2O$, 0,222 g $ZnSO_4.7\ H_2O$, 0,039 g $Na_2MoO_4.2\ H_2O$, 0,079 g $CuSO_4.5\ H_2O$ und 0,06 g $CoCl_2.6\ H_2O$. Die Zellpaste (150 g Naßgewicht) wird in 1 Volumen Puffer A (40 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 1200 bar aufgeschlossen. Anschließend wird Polymin P zugegeben und und der Niederschlag abgetrennt. Der Überstand wird mittels Dialyse gegen Puffer B (40 mmol/l Tris-HCl, pH 8,0, 0,1 mmol/l EDTA, 7 mmol/l 2-Mercaptoethanol, 10 % (V/V) Glycerin) entsalzt und an einer mit Puffer B vorgewaschenen Heparin-Sepharose-Säule fraktioniert. Zur Elution wird ein Gradient von 0 - 1 mol/l NaCl verwendet. DsaV wird in den Fraktionen zwischen 0,32 und 0,45 mol/l NaCl gefunden. Die aktiven Fraktionen werden gegen Puffer B dialysiert. Anschließend werden sie auf eine DEAE Sephacel®-Säule, welche mit Puffer B equilibriert wurde, aufgegeben. Zur Elution wird ein Gradient von 0 - 1 mol/l NaCl in Puffer B verwendet. DsaV wird in den Fraktionen zwischen 0,10 und 0,20 mol/l NaCl gefunden.

Die aktiven Fraktionen werden gegen Puffer B dialysiert und auf einer mit Puffer B äquilibrierten P 11-Säule fraktioniert. Zur Elution wird ein Gradient von 0 - 1 mol/l NaCl in Puffer B verwendet. DsaV wird in den Fraktionen zwischen 0,35 und 0,45 M NaCl gefunden. Die aktiven Fraktionen werden gegen Puffer C (10 mmol/l $K_2HPO_4/KH_2PO_4$, pH 8,0; 0,1 mmol/l EDTA; 7 mmol/l 2-Mercaptoethanol; 10 % (v/v) Glycerin) dialiysiert und auf einer mit Puffer (äquilibrierten Hydroxylapatit-Säule franktioniert. Zur Elution wird ein Gradient von 10 - 500 mmol/l $K_2HPO_4/KH_2PO_4$ im Puffer verwendet. DsaV wird in den Fraktionen zwischen 200 und 350 mmol Phosphat gefunden. Die aktiven Fraktionen werden zusammengegeben und gegen Lagerpuffer (20 mmol/l Tris-HCl, pH 7,5, 10 mmol/l 2-Mercaptoethanol und 100 mmol/l NaCl, 50% (v/v) Glycerin) dialysiert.

Beispiel 2

Bestimmung der Aktivität

Definition der Enzymeinheiten:

1 U DsaV spaltet 1 µg Lambda-dcm⁻-DNA innerhalb 1 Stunde bei 60 °C in 25 µl Endvolumen.

Zu einer Mischung von 2,5 µl Inkubationspuffer (330 mmol/l Tris-Acetat, pH 7,9/37 °C, 660 mmol/l Kaliumacetat, 100 mmol/l Magnesiumacetat und 5 mmol/l DTE werden 17,5 µl Wasser und 5 µl Lambda-dcm⁻-DNA (optische Dichte: 4 OD/ml) sowie 1 µl DsaV-Lösung (1 U/µl) zugegeben. Die Lösung wird eine Stunde bei 60 °C inkubiert, auf Eis gekühlt und mit 5 µl eines Abstopp-Reagenses, bestehend aus 7 mmol/l Harnstoff, 20 % (w/v) Saccharose, 60 mmol/l EDTA und 0,01 % (w/v Bromphenolblau versetzt. Anschließend wird eine Trennung durch Elektrophorese in 1 % Agarose-Gelen für 2 - 3 Stunden bei 100 V durchgeführt. Die erhaltenen Banden werden über Vergleich mit einem DNA-Längenstandard identifiziert.

**Ansprüche**

1. Typ II-Restriktionsendonuklease mit der Erkennungssequenz und der durch die Markierung charakterisierten Schnittstelle

5'-|CCNGG -3'
3'- GGNCC|-5'

2. Restriktionsendonuklease nach Anspruch 1, dadurch gekennzeichnet, daß sie aus Microorganismen der Gattung Dactylococcopsis erhältlich ist.

3. Restriktionsendonuklease nach Anspruch 1 oder 2, dadurch gekennzeichnet, daß sie aus Dactylococcopsis salina DSM 4080 erhältlich ist.

4. Restriktionsendonuklease nach den Ansprüchen 1 und 2, gekennzeichnet durch ein Temperatur-Optimum bei ca. 60 °C und ein pH-Optimum zwischen 7,6 und 8,2.

5. Verfahren zur Gewinnung einer TypII-Restriktionsendonuklease mit der Erkennungssequenz und der durch die Markierung charakterisierten Spaltstelle

5'-|CCNGG -3'
3'- GGNCC|-5'

dadurch gekennzeichnet, daß man Mikroorganismen der Gattung Dactylococcopsis züchtet und das Enzym aus den Zellen gewinnt.

6. Verfahren nach Anspruch 5, dadurch gekennzeichnet, daß man Dactylococcopsis salina DSM 4080 züchtet.

7. Verfahren nach den Ansprüchen 5 und 6, dadurch gekennzeichnet, daß man die Zellen aufschließt und den Zellüberstand gewinnt.

8. Verfahren nach Anspruch 7, dadurch gekennzeichnet, daß man zur Feinreinigung den Zellüberstand einer Affinitätschromatographie, einer Anionenaustauschchromatographie, einer Kationenaustauschchromatographie und einer abschließenden Chromatographie über Hydroxylapatit unterwirft.

9. Verfahren nach Anspruch 8, dadurch gekennzeichnet, daß man zur Affinitätschromatographie trägerfixiertes Heparin verwendet.

10. Verwendung einer TypII-Restriktionsendonuklease mit der Erkennungssequenz und der durch die Markierung charakterisierten Schnittstelle

5'-|CCNGG -3'
3'- GGNCC|-5'

ist, zur Erkennung und Spaltung der doppelsträngigen DNA-Sequenz 5'-CCNGG-3' und ihrer komplementären Sequenz.

Europäisches Patentamt

# EUROPÄISCHER RECHERCHENBERICHT

Nummer der Anmeldung

EP 90 11 3713

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | NUCLEIC ACIDS RESEARCH, Band 17, Supplement S, April 1989, Seiten 367,376-387; R.J. ROBERTS: "Restriction enzymes and their isoschizomers" * Seite 367, Zeile 24 * | 1 | C 12 N 9/22 |
| X | CHEMICAL ABSTRACTS, Band 103, Nr. 3, 22. Juli 1985, Seiten 246,247, Zusammenfassung Nr. 18801s, Columbus, Ohio, US; T.M. UPOROVA et al.: "Restriction endonucleases from Shigella sonnei 47", & VOPR. MED. KHIM., 31(2), 131-6 * Zusammenfassung * | 1 | |
| A | US-A-4 871 664 (BOEHRINGER MANNHEIM GmbH) * Das ganze Dokument * | 1-9 | |

RECHERCHIERTE SACHGEBIETE (Int. Cl.5)

C 12 N

Der vorliegende Recherchenbericht wurde für alle Patentansprüche erstellt

| Recherchenort | Abschlußdatum der Recherche | Prüfer |
|---|---|---|
| Den Haag | 19 November 90 | NAUCHE S.A. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument